# EUROPEAN PATENT SPECIFICATION

(11) **EP 4 644 363 B1**
(45) Date of publication and mention of the grant of the patent: **15.07.2026**
(21) Application number: 24919149.5
(22) Date of filing: 23.12.2024
(51) Int. Cl.: C07C 235/06, C07C 231/24

(54) **PURIFICATION METHOD FOR 3-METHOXY-N,N-DIMETHYLPROPIONAMIDE**
REINIGUNGSVERFAHREN FÜR 3-METHOXY-N,N-DIMETHYLPROPIONAMID
PROCÉDÉ DE PURIFICATION DE 3-MÉTHOXY-N,N-DIMÉTHYLPROPIONAMIDE

(30) Priority: 19.02.2024 CN 202410185181
(43) Date of publication of application: 05.11.2025
(73) Proprietor: Shenzhen Prechem New Materials Co., Ltd., Nanshan District Shenzhen, Guangdong 518055 (CN)
(72) Inventor: LAN, Junjie, Shenzhen, Guangdong 518055 (CN); WU, Xiaoqiang, Shenzhen, Guangdong 518055 (CN); YU, Donge, Shenzhen, Guangdong 518055 (CN)
(74) Representative: Metida
(86) International application number: PCT/CN2024/141613
(87) International publication number: WO 2025/175897

(56) References cited:
- CN-A- 106 966 923
- CN-A- 116 874 385
- CN-A- 116 924 909
- CN-A- 116 947 674
- CN-A- 117 776 957

## Description

### TECHNICAL FIELD

The present invention relates to the technical field of chemical materials, and in particular to a purification method of 3-methoxy-N,N-dimethylpropionamide (MMPA).

### BACKGROUND

3-methoxy-N,N-dimethylpropionamide (MMPA) is a colorless and transparent solvent having an amide group and an alkyl group. It is an aprotic solvent with a high boiling point and high polarity, and it can be mixed with a wide range of solvents and effectively dissolve polyamide. MMPA exhibits strong solvency, and has the characteristics of high permeability, low viscosity, low volatility, non-corrosiveness, high fluidity, and low surface tension. It is non-irritating to skin, safe and environmentally friendly. Therefore, it is an ideal substitute for traditional toxic solvents, such as N-Methyl-2-pyrrolidone, and is widely used in industries such as electronics, pharmaceuticals, pesticides, pigments, cleaning agents, and insulating materials. Nowadays, as the market for safe solvents becomes increasingly diversified and competitive, in addition to strong solvency, properties such as high volatility and low toxicity have become important competitive factors. Therefore, products have broad market prospects.

At present, MMPA on the market has a purity of 97%-98%. For example, Meryer (Shanghai) Biochemical Technology Co., Ltd. offers MMPA with a purity of 98%. CN116874385A discloses the following method for purification of MMPA: after completion of the reaction, excess dimethylamine was recovered and the catalyst was removed by centrifugation. The reaction mixture was concentrated and purified by distillation. The final purity of MMPA was 98.2%.

Although such purity may be acceptable for use in pesticides, pigments, cleaning agents and other industries, they are insufficient for some special fields, such as being used as a solvent for battery electrolytes cathode materials, where a purity of the solvent is required to reach 99.9% or higher to ensure optimal performance of the battery. However, the purity of most of the MMPA currently available fails to meet the requirement. As a result, toxic NMP is still predominantly used as the solvent. Although some patents have reported that high-purity MMPA (purity > 99.9%) had been obtained through improved synthetic processes, no high-purity MMPA products are commercially available on the market yet. This may be attributed to the fact that the improved synthetic processes are still limited to small-scale production and fail to meet the purity requirements after scale-up production. It may also be because the new synthetic processes have higher requirements for raw materials or equipment, making the scale-up production through the improved synthetic processes costly. At present, to obtain high-purity MMPA, focus is mainly placed on further optimizing purification processes, which can achieve better purification effects at lower cost. To date, there are no available reports on purification processes for MMPA. Therefore, further research into the MMPA purification processes is very necessary for the preparation of high-purity MMPA.

### SUMMARY

In order further improve the purity of 3-methoxy-N,N-dimethylpropionamide (MMPA) and broaden its application fields, the present invention provides a purification method of 3-methoxy-N,N-dimethylpropionamide.

A purification method of 3-methoxy-N,N-dimethylpropionamide adopts the following technical solution:
A purification method of 3-methoxy-N,N-dimethylpropionamide, including the following steps: performing adsorption, polymerization, nanofiltration, and distillation steps in sequence on crude MMPA to obtain purified MMPA; where in the polymerization step, an initiator is added to adsorbed MMPA, and polymerization is performed at a preset temperature; and after a reaction of polymerization is completed, the nanofiltration step is initiated.

Impurities such as N,N-dimethylpropionamide are present in the MMPA, which are difficult to be isolated and remove through distillation or filtration. In the purification method of the present invention, an initiator is added to induce polymerization of the impurities, thereby increasing their molecular weight, the impurities with high molecular weight are then removed by the nanofiltration step to separate them from the MMPA, thereby improving the purity of MMPA. Preferably, the adsorption step includes two processes: activated carbon adsorption and molecular sieve adsorption.

More preferably, specific steps of the molecular sieve adsorption includes: performing the activated carbon adsorption on the MMPA at a flow rate of 1-8 times a volume of the activated carbon per hour, passing through a molecular sieve at a flow rate of 1-8 times a volume of the molecular sieve per hour, and entering the subsequent nanofiltration step.

In the present invention, most of the impurities such as water, ammonium salts, methanol, and methyl acrylate in the crude MMPA can be removed through the activated carbon adsorption and molecular sieve adsorption, which prevents the impurities from interfering with the subsequent polymerization step.

Preferably, the initiator used in the polymerization step is one or more of an organic peroxide initiator, an inorganic peroxide initiator, an azo initiator, and a redox initiator; more preferably, the initiator is ammonium persulfate, benzoyl peroxide, 2,2'-azobis(2-methylpropionamidine) dihydrochloride, and sodium persulfate; and an amount of the initiator is 0.05-0.15% by mass of the crude MMPA.

Preferably, the preset temperature is 75-85 °C, and polymerization time is 1-3 h.

Preferably, in the nanofiltration step, a flow rate is controlled at 10-150 L/h, and a pressure is controlled at 0.01-0.1 MPa.

Preferably, the distillation is rectification under vacuum, a distillation temperature is controlled at 90-130 °C, and a vacuum degree is controlled at -0.85 - 0.1 MPa.

More preferably, in the distillation step, when a top temperature of a distillation column reaches 74-84 °C, a purity of distillate fraction is monitored, and collection of the distillate fraction is commenced when the purity of distillate fraction reaches 99.9%.

The present invention has the following beneficial effects:
1) The purification method of the present invention is relatively simple and easy to industrialize. It can increase the purity of MMPA to more than 99.9%, meeting the solvent standards for use in battery electrolyte and cathode material. This expands the application scope of MMPA, and helps to replace the toxic compound NMP, making it environmentally friendly and having great strategic significance for the development of the battery industry.
2) In the purification method of the present invention, an adsorption step is first performed to remove impurities such as water, ammonium salts, methanol, and methyl acrylate, which avoids interference with the subsequent polymerization step. Moreover, an initiator is introduced in the polymerization step to cause N,N-dimethylpropionamide-type impurities to undergo polymerization reaction, increasing their molecular weight and forming compounds with high molecular weight or polymer gels, which are then removed by a nanofiltration step; and finally, further purification is performed by a distillation step to obtain high-purity MMPA. The process flow of the present invention is reasonably designed and can efficiently purify the MMPA.

### BRIEF DESCRIPTION OF THE DRAWINGS

FIG. 1 is a gas chromatogram of a purified MMPA product in Example 1.

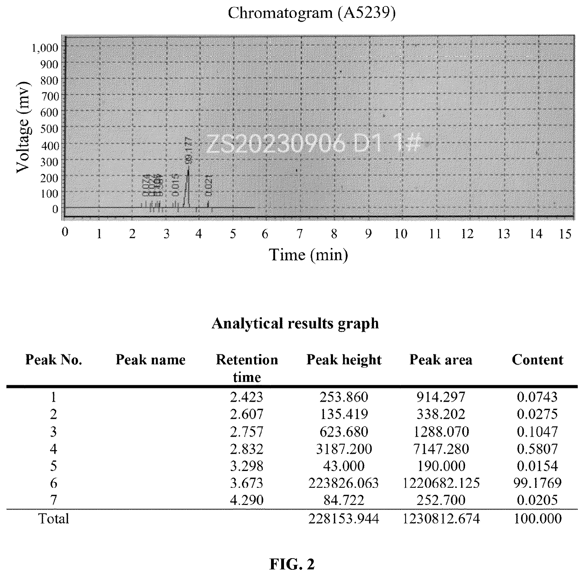

FIG. 2 is a gas chromatogram of a purified MMPA product in Comparative Example 1.
FIG. 3 is a gas chromatogram of a purified MMPA product in Comparative Example 2.
FIG. 4 is a gas chromatogram of a purified MMPA product in Example 2.

### DETAILED DESCRIPTION OF EMBODIMENTS

The present invention will be further described in detail below with reference to specific embodiments and accompanying drawings. The following embodiments are provided solely for further illustration of the present invention, and should not be construed as limiting the scope of the present invention.

In the present invention, the purity of MMPA is determined using gas chromatography (GC).

### Example 1

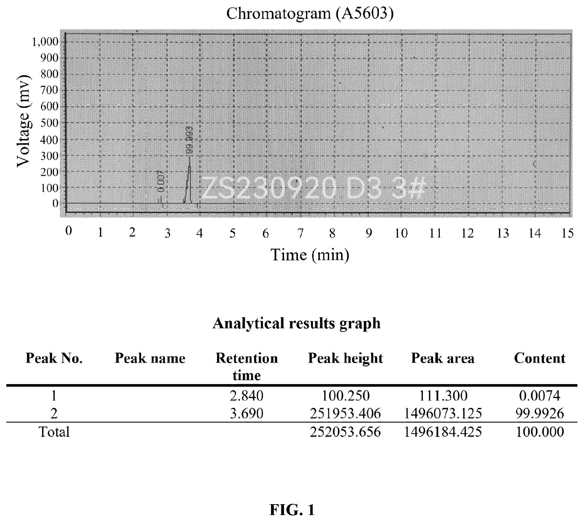

400 g of crude MMPA (with a purity of 98.15%) was weighed; activated carbon adsorption was first performed at a flow rate of 6 times a volume of the activated carbon per hour; molecular sieve adsorption was then performed at a flow rate of 5 times of a volume of molecular sieve per hour (the molecular sieve adsorption can remove impurities such as water, ammonium salts, methanol, and methyl acrylate in an MMPA solvent.); and the MMPA after the molecular sieve adsorption was introduced into a polymerization reactor. 0.4 g of ammonium persulfate was added to the polymerization reactor as an initiator; polymerization was conducted at 80 °C for 2 h to obtain a mixture; after the polymerization was completed, the mixture was transferred to a nanofiltration device; nanofiltration was performed at a flow rate of 80 L/h and a pressure of 0.01-0.1 MPa (the nanofiltration can remove compounds with high molecular weight and residual polymer colloids). The MMPA after the nanofiltration was introduced into a distillation kettle for rectification under vacuum; vacuum was slowly drawn until the vacuum reached a gauge pressure of -0.095 MPa, and front fraction was removed under a stable vacuum state, in which case, a kettle bottom temperature was 90 °C, and a top temperature was 60 °C; the temperature was slowly raised (at a heating rate of 0.5 °C/min), when the kettle bottom temperature reached 100 °C, and the top temperature reached 76-80 °C, distillate fraction were continuously monitored (once every 10 min). When the distillate fraction 99.9% was detected, collection of a finished product was commenced and continued until the end. A total of 382.65 g of product was obtained, and a final detected product content was 99.99%. The gas chromatogram is shown in FIG. 1.

### Comparative Example 1

This Comparative Example is basically the same as Example 1, except that no polymerization step was performed in this Comparative Example; that is, the MMPA obtained after nanofiltration was directly subjected to rectification under vacuum. Specific operation is as follows:
400 g of crude MMPA (with a purity of 98.15%) was weighed; activated carbon adsorption was first performed at a flow rate of 6 times a volume of the activated carbon per hour; molecular sieve adsorption was then performed at a flow rate of 5 times of a volume of molecular sieve per hour (the molecular sieve adsorption can remove impurities such as water, ammonium salts, methanol, and methyl acrylate in an MMPA solvent.); the MMPA after the molecular sieve adsorption was introduced into a nanofiltration device, nanofiltration was performed at a flow rate of 80 L/h and a pressure of 0.01-0.1 MPa (the nanofiltration can remove compounds with high molecular weight and residual polymer colloids); the MMPA after the nanofiltration was introduced into a distillation kettle for rectification under vacuum, vacuum was slowly drawn until the vacuum reached a gauge pressure of -0.095 MPa, and front fraction was removed under a stable vacuum state, in which case, a kettle bottom temperature was 90 °C, and a top temperature was 60 °C; the temperature was slowly raised (at a heating rate of 0.5 °C/min), when the kettle bottom temperature reached 100 °C, and the top temperature reached 76-80 °C, distillate fraction were continuously monitored (once every 10 min), and the detection was continuous for 1 h, but the purity of the fraction was difficult to reach 99.9%. Therefore, collection of a finished product was commenced and continued until the end when the distillate fraction reached a purity of 99.3%. A total of 385.95 g of product was obtained, and a final detected product content was 99.78%.

Compared with Comparative Example 1, Example 1 has a higher purity of MMPA, this may be because no polymerization reaction was performed, impurities such as N,N dimethylpropionamide were difficult to remove, making the purity difficult to be further improved.

### Comparative Example 2

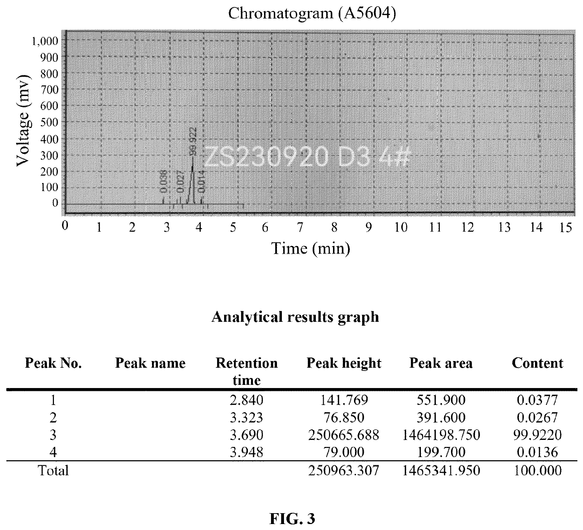

This Comparative Example is basically the same as Example 1, except that no molecular sieve adsorption step was performed carried out in this Comparative Example; that is, the crude was directly polymerized. Specific operation is as follows:
400 g of MMPA (with a purity of 98.15%) was weighed and directly added to a polymerization reactor, and 0.4 g of ammonium persulfate was added to the polymerization reactor as an initiator; polymerization was conducted at 80 °C for 2 h to obtain a mixture; after the polymerization was completed, the mixture was transferred to a nanofiltration device; nanofiltration was performed at a flow rate of 80 L/h and a pressure of 0.01-0.1 MPa (the nanofiltration can remove compounds with high molecular weight and residual polymer colloids). The MMPA after the nanofiltration was introduced into a distillation kettle for rectification under vacuum; vacuum was slowly drawn until the vacuum reached a gauge pressure of -0.095 MPa, and front fraction was removed under a stable vacuum state, in which case, a kettle bottom temperature was 90 °C, and a top temperature was 60 °C; the temperature was slowly raised (at a heating rate of 0.5 °C/min), when the kettle bottom temperature reached 100 °C, and the top temperature reached 76-80 °C, distillate fraction were continuously monitored (once every 10 min). When the distillate fraction 99.9% was detected, collection of a finished product was commenced and continued until the end. A total of 383.14g of product was obtained, and a final detected product content was 99.92%. The gas chromatogram is shown in FIG. 3.

Compared with Comparative Example 2, Example 1 has a higher purity of MMPA, which may be attributed to the exclusion of polymerization reaction. The adsorption effectively removed some impurities, thereby reducing interference with the polymerization reaction and resulting in a higher final purity.

### Example 2

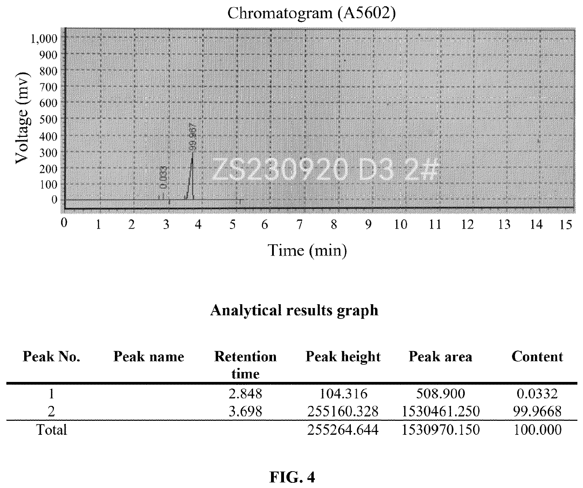

400 g of crude MMPA (with a purity of 97.80%) was weighed. First, activated carbon adsorption was performed at a flow rate of 6 times a volume of the activated carbon per hour; molecular sieve adsorption was then performed at a flow rate of 5 times of a volume of molecular sieve per hour (molecular sieve adsorption step could remove impurities such as water, ammonium salts, methanol, and methyl acrylate in an MMPA solvent.); and the MMPA after the molecular sieve adsorption was introduced into a polymerization reactor. 0.2 g of sodium persulfate was added to the polymerization reactor as an initiator; polymerization was conducted at 75°C for 2.5 h to obtain a mixture; after the polymerization was completed, the mixture was transferred to a nanofiltration device; nanofiltration was performed at a flow rate of 50 L/h and a pressure of 0.01-0.1 MPa (the nanofiltration can remove compounds with high molecular weight and residual polymer colloids). The MMPA after the nanofiltration was introduced into a distillation kettle for rectification under vacuum; vacuum was slowly drawn until the vacuum reached a gauge pressure of -0.095 MPa, and front fraction was removed under a stable vacuum state, in which case, a kettle bottom temperature was 92°C, and a top temperature was 63°C; the temperature was slowly raised (at a heating rate of 1°C/min), when the kettle bottom temperature reached 100 °C, and the top temperature reached 78-82°C, distillate fraction were continuously monitored (once every 10 min). When the distillate fraction with a purity of 99.9% was detected, collection of a finished product was commenced and continued until the end. A total of 380.65g of product was obtained, and a final detected product content was 99.96%. The gas chromatogram is shown in FIG. 4.

### Example 3

400 g of crude MMPA (with a purity of 98.02%) was weighed. First, activated carbon adsorption was performed at a flow rate of 7 times a volume of the activated carbon per hour; molecular sieve adsorption was then performed at a flow rate of 5 times of a volume of molecular sieve per hour (molecular sieve adsorption step could remove impurities such as water, ammonium salts, methanol, and methyl acrylate in an MMPA solvent.); and the MMPA after the molecular sieve adsorption was introduced into a polymerization reactor. 0.6 g of 2,2'-azobis(2-methylpropionamidine) dihydrochloride was added to the polymerization reactor as an initiator; polymerization was conducted at 75°C for 1.5 h to obtain a mixture; after the polymerization was completed, the mixture was transferred to a nanofiltration device; nanofiltration was performed at a flow rate of 100 L/h and a pressure of 0.01-0.1 MPa (the nanofiltration can remove compounds with high molecular weight and residual polymer colloids). The MMPA after the nanofiltration was introduced into a distillation kettle for rectification under vacuum; vacuum was slowly drawn until the vacuum reached a gauge pressure of -0.095 MPa, and front fraction was removed under a stable vacuum state, in which case, a kettle bottom temperature was 88°C, and a top temperature was 59°C; the temperature was slowly raised (at a heating rate of 1°C/min), when the kettle bottom temperature reached 98°C, and the top temperature reached 74-78 °C, distillate fraction were continuously monitored (once every 10 min). When the distillate fraction 99.9% was detected, collection of a finished product was commenced and continued until the end. A total of 384.26g of product was obtained, and a final detected product content was 99.97%.

## Claims

1. A purification method of 3-methoxy-N,N-dimethylpropionamide (MMPA), **characterized by** comprising the following steps:
performing adsorption, polymerization, nanofiltration, and distillation steps in sequence on crude MMPA to obtain purified MMPA; **characterized in that** in the polymerization step, an initiator is added to the MMPA after the adsorption step, and polymerization is performed at a preset temperature; and after a reaction of polymerization is completed, the nanofiltration step is initiated;
the adsorption is molecular sieve adsorption, with steps as follows: performing activated carbon adsorption on the crude MMPA at a flow rate of 6-7 times a volume of the activated carbon per hour, then passing through a molecular sieve at a flow rate of 5 times a volume of the molecular sieve per hour, and entering the subsequent nanofiltration step;
in the polymerization step, the initiator is one or more of ammonium persulfate, 2,2'-azobis(2-methylpropionamidine) dihydrochloride, and sodium persulfate; an amount of the initiator is 0.05-0.15% by mass of the crude MMPA; and the preset temperature is 75-80 °C, and polymerization time is 1-2.5 h; and
in the distillation step, the distillation is rectification under vacuum, a distillation temperature is controlled at 90-130 °C, and a vacuum degree of the distillation is controlled at -0.85 - 0.1 MPa during distillation; and when a top temperature of a distillation column reaches 74-84 °C, a purity of distillate fraction is monitored, and collection of the distillate fraction is commenced when the purity of distillate fraction reaches 99.9%.

2. The purification method of 3-methoxy-N,N-dimethylpropionamide according to claim 1, **characterized in that** in the nanofiltration step, a flow rate is controlled at 10-150 L/h, and a pressure is controlled at 0.01-0.1 MPa.

## Patentansprüche

1. Ein Reinigungsverfahren für 3-Methoxy-N,N-dimethylpropionamid (MMPA), **dadurch gekennzeichnet, dass** es die folgenden Schritte umfasst:
nacheinander Ausführen von Adsorptions-, Polymerisations-, Nanofiltrations- und Destillationsschritten an rohem MMPA, um gereinigtes MMPA zu erhalten; **dadurch gekennzeichnet, dass** in dem Polymerisationsschritt dem MMPA nach dem Adsorptionsschritt ein Initiator zugesetzt wird und die Polymerisation bei einer voreingestellten Temperatur durchgeführt wird; und nachdem eine Polymerisationsreaktion abgeschlossen ist, der Nanofiltrationsschritt eingeleitet wird;
die Adsorption eine Molekularsiebadsorption ist, mit den folgenden Schritten: Durchführen einer Aktivkohleadsorption an dem rohen MMPA mit einer Durchflussrate des 6- bis 7-Fachen eines Volumens der Aktivkohle pro Stunde, dann Leiten durch ein Molekularsieb mit einer Durchflussrate des 5-Fachen eines Volumens des Molekularsiebs pro Stunde und Eintreten in den nachfolgenden Nanofiltrationsschritt;
in dem Polymerisationsschritt der Initiator einer oder mehrere von Ammoniumpersulfat, 2,2'-Azobis(2-methylpropionamidin)dihydrochlorid und Natriumpersulfat ist; eine Menge des Initiators 0.05 bis 0.15 Massen-% des rohen MMPA beträgt; und die voreingestellte Temperatur 75-80 °C beträgt und die Polymerisationszeit 1-2.5 h beträgt; und
in dem Destillationsschritt die Destillation eine Rektifikation unter Vakuum ist, eine Destillationstemperatur auf 90-130 °C geregelt wird und ein Vakuumgrad der Destillation während der Destillation auf -0,85 bis 0,1 MPa geregelt wird; und wenn eine Kopftemperatur einer Destillationskolonne 74-84 °C erreicht, eine Reinheit der Destillatfraktion überwacht wird und die Sammlung der Destillatfraktion begonnen wird, wenn die Reinheit der Destillatfraktion 99.9 % erreicht.

2. Das Reinigungsverfahren für 3-Methoxy-N,N-dimethylpropionamid nach Anspruch 1, **dadurch gekennzeichnet, dass** in dem Nanofiltrationsschritt eine Durchflussrate auf 10-150 L/h geregelt wird und ein Druck auf 0.01-0.1 MPa geregelt wird.

## Revendications

1. Un procédé de purification de 3-méthoxy-N,N-diméthylpropionamide (MMPA), **caractérisé en ce qu'**il comprend les étapes suivantes:
la réalisation d'étapes d'adsorption, de polymérisation, de nanofiltration et de distillation en séquence sur du MMPA brut pour obtenir du MMPA purifié;
**caractérisé en ce que**, dans l'étape de polymérisation, un initiateur est ajouté au MMPA après l'étape d'adsorption, et la polymérisation est effectuée à une température prédéfinie; et après l'achèvement d'une réaction de polymérisation, l'étape de nanofiltration est initiée;
l'adsorption est une adsorption sur tamis moléculaire, avec les étapes suivantes: la réalisation d'une adsorption sur charbon actif sur le MMPA brut à un débit de 6 à 7 fois un volume du charbon actif par heure, puis le passage à travers un tamis moléculaire à un débit de 5 fois un volume du tamis moléculaire par heure, et l'entrée dans l'étape de nanofiltration subséquente;
dans l'étape de polymérisation, l'initiateur est un ou plusieurs éléments parmi le persulfate d'ammonium, le dichlorhydrate de 2,2'-azobis(2-méthylpropionamidine) et le persulfate de sodium; une quantité de l'initiateur est de 0.05 à 0.15 % en masse du MMPA brut; et la température prédéfinie est de 75 à 80 °C, et un temps de polymérisation est de 1 à 2.5 h; et
dans l'étape de distillation, la distillation est une rectification sous vide, une température de distillation est contrôlée à 90-130 °C, et un degré de vide de la distillation est contrôlé à -0,85 - 0,1 MPa pendant la distillation; et lorsqu'une température de tête d'une colonne de distillation atteint 74-84 °C, une pureté de fraction de distillat est surveillée, et la collecte de la fraction de distillat commence lorsque la pureté de la fraction de distillat atteint 99.9 %.

2. Le procédé de purification de 3-méthoxy-N,N-diméthylpropionamide selon la revendication 1, **caractérisé en ce que**, dans l'étape de nanofiltration, un débit est contrôlé à 10-150 L/h, et une pression est contrôlée à 0.01-0.1 MPa.
